# EUROPÄISCHE PATENTSCHRIFT

(11) **EP 1 569 724 B1**
(45) Veröffentlichungstag und Bekanntmachung des Hinweises auf die Patenterteilung: **31.12.2008**
(21) Anmeldenummer: 03775265.6
(22) Anmeldetag: 30.10.2003
(51) Int. Cl.: A61P 17/14, A61K 47/10, A61K 9/00, A61K 31/4045, A61K 31/4188, A61K 36/16, A61K 47/46

(54) **FORMULIERUNGEN ENTHALTEND MELATONIN, GINKGO BILOBA UND BIOTIN**
FORMULATIONS CONTAINING MELATONIN, GINKGO BILOBA, AND BIOTIN
FORMULATIONS CONTENANT DE LA MELATONINE, DU GINKGO BILOBA ET DE LA BIOTINE

(30) Priorität: 30.10.2002 DE 10250646; 18.11.2002 DE 20217814 U; 29.01.2003 US 353056
(43) Veröffentlichungstag der Anmeldung: 07.09.2005
(73) Patentinhaber: Asat AG Applied Science & Technology, 6300 Zug (CH)
(72) Erfinder: SCHMID, Hans, W., CH-6303 Zug (CH)
(74) Vertreter: Weiss, Wolfgang
(86) Internationale Anmeldenummer: PCT/EP2003/012097
(87) Internationale Veröffentlichungsnummer: WO 2004/039454

(56) Entgegenhaltungen:
- WO-A-03/049687
- GB-A- 2 370 504
- US-A- 5 750 107
- US-A- 6 013 279
- US-A- 6 030 948
- US-A1- 2002 028 257
- US-A1- 2002 061 870
- US-A1- 2002 098 253
- US-A1- 2002 182 196
- DATABASE WPI Section Ch, Week 199134 Derwent Publications Ltd., London, GB; Class B01, AN 1991-248680 XP002270609 & JP 03 161426 A (KOBAYASH KOSE KK) 11. Juli 1991 (1991-07-11)
- KOBAYASHI N ET AL: "EFFECT OF LEAVES OF GINKGO BILOBA ON HAIR REGROWTH IN C3H STRAIN MICE" PHARMACEUTICAL SOCIETY OF JAPAN. JOURNAL - YAKUGAKU ZASSHI, PHARMACEUTICAL SOCIETY OF JAPAN, TOKYO, JP, Bd. 113, Nr. 10, 1. Oktober 1993 (1993-10-01), Seiten 718-724, XP000607032 ISSN: 0031-6903

## Beschreibung

Die vorliegende Erfindung betrifft eine Zusammensetzung, die als Wirkstoffe eine Kombination von Melatonin, Ginkgo Biloba und Biotin enthält. Die Zusammensetzung eignet sich insbesondere zur Herstellung von Formulierungen für die topische Anwendung im Haar.

Die Verwendung von Melatonin zur positiven Beeinflussung des Haarwachstums ist bekannt. Melatonin besitzt dank der selektiven Interaktion mit proliferierenden Zellen der Haarfollikel, welche das Wachstum der Haare steuern, eine stimulierende Wirkung auf das Haarwachstum. Diese stimulierende Wirkung kann bei der Verwendung verschiedener Konzentrationen an Melatonin beobachtet werden.

US-Patent 5,952,373 beschreibt ein Verfahren zur Hautbehandlung, bei dem eine Zusammensetzung aufgebracht wird, die als Wirkstoff ein oder mehrere Flavonoide enthält. Es findet sich kein Hinweis auf die Verwendung von Melatonin als Wirkstoff zur Verbesserung des Haarwachstums.

US-Patentanmeldung US 2002/0061870 offenbart eine Zusammensetzung zur Linderung von Gehörverlust oder Tinnitus. Diese Zusammensetzung, die Form einer Tablette verabreicht wird, enthält eine Vielzahl von Wirkstoffen, darunter gegebenenfalls auch Melatonin, Biotin oder Gingko Biloba. Ein Hinweis auf die Verwendung zur Verbesserung des Haarwachstums bzw. auf eine topische Applikation findet sich nicht.

Kobayashi et al. beschreiben die Wirkung eines Ginkgo-Extrakts auf das Haarwachstum in Mäusen (Kobayashi Noboru; Suzuki Ruka; Koide Chiharu; Suzuki Tadashi; Matsuda Hideaki; Kubo Michinori; "Effect of leaves of Ginkgo biloba on hair regrowth in C3H strain mice"; Yakugaku Zasshi; 113(10) 718-724; 1993.

US-Patent 5,750,107 beschreibt ein topisches Haarwachsmittel, welches Pflanzenextrakt und Biotin enthält.

US-Patent 6,030,948 offenbart eine topische Zusammensetzung die Melatonin enthält, zur Behandlung von Alopezie.

Überraschenderweise wurde im Rahmen der zur vorliegenden Erfindung führenden Untersuchungen festgestellt, dass der Zusatz von Ginkgo Biloba und Biotin bei gleichzeitiger Anwendung mit Melatonin die Wirkung von Melatonin verstärken und die Retention von Melatonin auf der Kopfhaut-Oberfläche erhöht wird.

Ein Gegenstand der vorliegenden Erfindung ist somit eine Zusammensetzung, die als Wirkstoffe (a) Melatonin oder ein Derivat davon, (b) Ginkgo Biloba als Extrakt oder/und einen oder mehrere Inhaltsstoffe davon und (c) Biotin enthält. Die Zusammensetzung ist vorzugsweise eine topische Formulierung, die für pharmazeutische oder/und kosmetische Anwendungen, insbesondere zum Aufbringen auf die Kopfhaut, geeignet ist.

Die erfindungsgemäße Zusammensetzung enthält eine Kombination von Wirkstoffen. Die erste Komponente dieser Kombination ist Melatonin oder ein Melatoninderivat. Melatoninderivate werden vorzugweise ausgewählt aus 5-Methoxytryptamin, 5-Methoxytryptophan, 5-Methoxytryptophol, 5-Methoxyindol-3-essigsäure und 6-Hydroxy-Melatonin. Neben diesen Substanzen können auch physiologisch akzeptable Salze, Ester und Komplexverbindungen davon eingesetzt werden.

Die zweite Komponente der Zusammensetzung ist Ginkgo Biloba. Ginkgo Biloba besitzt einen positiven Einfluss auf verschiedene Stoffwechselfunktionen. Eine weitere vorteilhafte Eigenschaft von Ginkgo ist die Verstärkung des Radikalfängerpotenzials. Bevorzugt wird ein Ginkgo Biloba als Extrakt, insbesondere als Trockenextrakt, oder/und ein oder mehrere Inhaltsstoffe davon verwendet. Besonders geeignet zur Verwendung der erfindungsgemäßen Zusammensetzung sind Trockenextrakte aus Ginkgo-Blättern.

Eine weitere Komponente der erfindungsgemäßen Zusammensetzung ist Biotin. Biotin entfaltet seine Wirkung beispielsweise als Coenzym im Fettsäuren- und Aminosäurenstoffwechsel, z.B. bei Transcarboxylierungsreaktionen.

Die Konzentration der Wirkstoffe in der Zusammensetzung kann - je nach Anwendung - in breiten Bereichen variiert werden. Günstigerweise liegen die Konzentrationen der Wirkstoffe jeweils unabhängig im Bereich zwischen 0,0001 % (Gewicht) bis 1 % (Gewicht) bezogen auf das Gesamtgewicht der Zusammensetzung. Die Konzentration von Melatonin oder einem Melatoninderivat ist vorzugsweise 0,001 % (Gewicht) bis 0,01 % (Gewicht). Die Konzentration von Ginkgo Biloba ist vorzugsweise 0,01 % (Gewicht) bis 0,1 % (Gewicht). Die Konzentration von Biotin ist vorzugsweise 0,002 % (Gewicht) bis 0,05 % (Gewicht).

Pro Applikation werden bevorzugt etwa 0,001 mg bis etwa 10 mg, bevorzugt etwa 0,01 mg bis etwa 1 mg Melatonin bzw. Melatonin-Derivat verabreicht.

Die drei Wirkstoff-Komponenten der erfindungsgemäßen Zusammensetzung entwickeln ein Wirkungsprofil, welches das Haarwachstum fördert, einen Alterungsschutz bereitstellt, die Haardicke verbessert oder/und die Telogenrate reduziert. Die Melatoninwirkung am Haarfollikel wird durch die Kombination mit Ginkgo Biloba und Biotin überraschenderweise verstärkt. Somit können auch sehr niedrige Konzentrationen an Melatonin, beispielsweise etwa 0,001-10 mg, bevorzugt etwa 0,01-1 mg pro Applikation, mit zufriedenstellender Wirkung eingesetzt werden.

Die Kombination von Melatonin, Ginkgo Biloba und Biotin kann in einem geeigneten Trägersystem verabreicht werden. Vorzugsweise sind die Wirkstoffe gelöst oder/und dispergiert in einem flüssigen, halbfesten oder festen Trägersystem enthalten. Beispiele für geeignete Trägersysteme sind Flüssigkeiten wie Wasser oder wässrige Pufferlösungen, physiologisch verträgliche organische Lösungsmittel wie Ethanol oder Kombinationen davon, Öl-Wasser-Emulsionen, Wasser-Öl-Emulsionen, Fette, Polyethylenglykole, Propylenglykole, Glycerin, Emulgatoren oder Kombinationen davon sowie andere in pharmazeutischen und kosmetischen Formulierungen verwendete Träger- oder Hilfsstoffe.

Die Verwendung eines wässrigen Trägersystems, d.h. eines Trägersystems mit einem Wassergehalt von 50 % (Gewicht) oder mehr, insbesondere von 60 % (Gewicht) oder mehr, ist bevorzugt. Der pH-Wert eines wässrigen Trägersystems wird vorzugsweise in einem Bereich zwischen pH 3 und pH 6, besonders bevorzugt im Bereich von pH 3,5 und pH 4, eingestellt.

Diese Trägersysteme ermöglichen vorteilhafterweise eine gezielte Wirkstoffabgabe an das Haarfollikel. Dadurch wird die Wirkstoffplatzierung im Haarfollikel optimiert. Weiterhin kann durch das Trägersystem eine gesteuerte Absorption der Wirkstoffe Melatonin, Ginkgo Biloba und Biotin ermöglicht werden.

Die Verarbeitung von Melatonin mit Ginkgo Biloba und Biotin in geeigneten Trägersystemen kann darüber hinaus zu einer Verhinderung oder Verzögerung der Melatoninresorption führen. Dadurch wird der normale humane Plasmaspiegel nicht beeinflusst. Zudem kann eine länger anhaltende Wirkung der Wirkstoffe erzielt werden.

Durch Formulierung in einem Trägersystem können stabile Formulierungen erhalten werden, die in der Kosmetik, insbesondere in Form kosmetischer Lösungen, verwendet werden.

Bei Verwendung von speziellen Formulierungssystemen, wie Liposomen, Nanosomen oder festen Einschlussträgern, wie Agarose, können Zusammensetzungen erhalten werden, die eine kontrollierte Abgabe von zumindest einem der Wirkstoffe, insbesondere von allen Wirkstoffen, erlauben.

Die erfindungsgemäßen Zusammensetzungen eignen sich insbesondere für pharmazeutische oder/und kosmetische Anwendungen beispielsweise im Haar. Die Zusammensetzungen können als Lösung, Suspension, Emulsion, Mikroemulsion, Nanosystem, Creme, Gel, Lotion, Spray, Schaum oder Salbe oder in jeder anderen für topische Anwendungen geeigneten Form vorliegen. Sie werden überlicherweise in einem Verpackungs- oder Anwendungssystem ausgewählt aus Tuben, Flaschen, Sprayflaschen, Pflastern, Schwämmen und Textil- oder Kunststoffträgern und anderen für die Applikation im Haar geeigneten Systemen eingesetzt. Ein besonders bevorzugtes Verpackungssystem sind Eindosis-Ampullen, welche eine Dosierungseinheit für eine Applikation bereitstellen. Die Ampullen können aus verschiedenen Materialien, z.B. aus Glas oder Kunststoff, gefertigt sein. Besonders bevorzugt sind Kunststoffampullen, da Kunststoffampullen eine sichere und gute Handhabung gewährleisten.

Neben den Wirkstoffen können die erfindungsgemäßen Zusammensetzungen einen oder mehrere kosmetische oder/und pharmazeutische Hilfs- bzw. Zusatzstoffe, beispielsweise Verdickungsmittel, Mineralstoffe, Öle, Vitamine, z.b. Vitamin A, insbesondere in Form der Retinsäure, oder Duftstoffe, enthalten.

Die erfindungsgemäßen Kombinationspräparate werden insbesondere zur Anwendung am Abend empfohlen und entfalten ihre Wirkung insbesondere während der Nachtstunden. Bei dieser Art von Anwendung ist die Wirkung der Zusammensetzung besonders stark. Die erfindungsgemäßen Zusammensetzungen eignen sich insbesondere zur Stimulierung der Haarfollikel und einer positiven Beeinflussung des Haarwachstums.

Die Behandlung erfolgt insbesondere in topischen, für die Anwendung im Haar geeigneten Applikationen, welche die entsprechenden, für den jeweiligen Verwendungszweck geeigneten Konzentrationen der Wirkstoffe enthalten.

Die erfindungsgemäße Zusammensetung kann zur Förderung des Haarwachstums eingesetzt werden, insbesondere zur Prävention oder/und Behandlung von Alopezie bei Männern oder bei Frauen. Besonders bevorzugte Indikationen sind die androgenetische Alopezie des männlichen Typs, die androgenetische Alopezie des weiblichen Typs, die diffuse Alopezie des männlichen Typs und die diffuse Alopezie des weiblichen Typs.

Die Erfindung wird weiterhin durch das folgende Beispiel erläutert.

### Beispiel: Zusammensetzung einer erfindungsgemäßen Formulierung mit den Wirkstoffen Melatonin, Ginkgo Biloba und Biotin

Die Zusammensetzung enthält 0,05 Gew.-% Ginkgo Biloba Trockenextrakt, 0,01 Gew.-% Biotin, 0,0033 Gew.-% Melatonin sowie weitere Zusatzstoffe, Wasser und Ethanol. Der pH-Wert der Zusammensetzung beträgt zwischen 3,5 und 4.

## Patentansprüche

1. Zusammensetzung als topische Formulierung zur Anwendung im Haar,
**dadurch gekennzeichnet,**
**dass** sie als Wirkstoffe
(a) Melatonin oder ein Derivat davon,
(b) Ginkgo Biloba und
(c) Biotin enthält,
wobei das Melatonin-Derivat ausgewählt wird aus 5-Methoxytryptamin, 5-Methoxytryptophan, 5-Methoxytryptophol, 5-Methoxyindol-3-essigsäure und 6-Hydroxy-melatonin sowie physiologisch akzeptable Salze, Ester und Komplexverbindungen davon

2. Zusammensetzung nach Anspruch 1,
**dadurch gekennzeichnet,**
**dass** sie Ginkgo Biloba als Trockenextrakt oder/und einen oder mehrere Inhaltsstoffe davon enthält.

3. Zusammensetzung nach einem der vorhergehenden Ansprüche,
**dadurch gekennzeichnet,**
**dass** die Konzentration oder Wirkstoffe jeweils im Bereich zwischen 0,0001 % (Gewicht) und 1 % (Gewicht) bezogen auf das Gesamtgewicht der Zusammensetzung liegt.

4. Zusammensetzung nach einem der vorhergehenden Ansprüche,
**dadurch gekennzeichnet,**
**dass** die Wirkstoffe gelöst oder/und dispergiert in einem flüssigen, halbfesten oder festen Trägersystem vorliegen.

5. Zusammensetzung nach einem der vorhergehenden Ansprüche,
**dadurch gekennzeichnet,**
**dass** sie eine kontrollierte Abgabe von zumindest einem der Wirkstoffe erlaubt.

6. Zusammensetzung nach einem der vorhergehenden Ansprüche für pharmazeutische oder/und kosmetische Anwendungen.

7. Zusammensetzung nach Anspruch 1,
**dadurch gekennzeichnet,**
**dass** die transdermale Absorption von zumindest einem der Wirkstoffe steuerbar ist.

8. Zusammensetzung nach einem der vorhergehenden Ansprüche als Lösung, Suspension, Emulsion, Mikroemulsion, Nanosystem, Creme, Gel, Lotion, Spray, Schaum oder Salbe.

9. Zusammensetzung nach einem der vorhergehenden Ansprüche in einem Verpackungs- oder Anwendungssystem ausgewählt aus Eindosis-Ampullen aus Kunststoff oder Glas, Tuben, Flaschen, Sprayflaschen, Pflastern, Schwämmen und Textil- oder Kunststoffträgern.

10. Zusammensetzung nach einem der vorhergehenden Ansprüche zur Verstärkung der Haarfollikel-stimulierenden Wirkung von Melatonin durch den Zusatz von Ginkgo Biloba und Biotin.

11. Verwendung einer Zusammensetzung nach einem der vorhergehenden Ansprüche zur zur Herstellung eines Mittels für die Förderung des Haarwachstums.

12. Verwendung einer Zusammensetzung nach einem der vorhergehenden Ansprüche zur Herstellung eines Mittels für die Prävention oder/und Behandlung von Alopezie.

13. Verwendung nach Anspruch 12 zur Behandlung von Alopezie bei Männern.

14. Verwendung nach Anspruch 12 zur Behandlung von Alopezie bei Frauen.

15. Verwendung nach einem der Ansprüche 11 bis 14.
**dadurch gekennzeichnet,**
**dass** pro Applikation 0,001 mg bis 10 mg Melatonin verabreicht werden.

16. Verwendung nach Anspruch 15,
**dadurch gekennzeichnet,**
**dass** pro Applikation 0,01 mg bis bis 1 mg Melatonin verabreicht werden.

## Claims

1. Composition as topical formulation for use in the hair,
**characterized in that**
it comprises as active ingredients
(a) melatonin or a derivative thereof,
(b) ginkgo biloba and
(c) biotin,
the melatonin derivative being selected from 5-methoxytryptamine, 5-methoxytryptophan, 5-methoxytryptophol, 5-methoxyindole-3-acetic acid and 6-hydroxymelatonin, and physiologically acceptable salts, esters and complex compounds thereof.

2. Composition according to claim 1,
**characterized in that**
it comprises ginkgo biloba as dry extract or/and one or more constituents thereof.

3. Composition according to any of the preceding claims,
**characterized in that**
the concentration of the active ingredients is in each case in the range between 0.0001% (by weight) and 1% (by weight) based on the total weight of the composition.

4. Composition according to any of the preceding claims,
**characterized in that**
the active ingredients are present as solution or/ and dispersion in a liquid, semisolid or solid carrier system.

5. Composition according to any of the preceding claims,
**characterized in that**
it permits controlled delivery of at least one of the active ingredients.

6. Composition according to any of the preceding claims for pharmaceutical or/and cosmetic applications.

7. Composition according to claim 1,
**characterized in that**
the transdermal absorption of at least one of the active ingredients is controllable.

8. Composition according to any of the preceding claims as solution, suspension, emulsion, microemulsion, nanosystem, cream, gel, lotion, spray, foam or ointment.

9. Composition according to any of the preceding claims in a packaging or application system selected from single-dose ampules made of plastics or glass, tubes, bottles, spray bottles, patches, sponges and textile or plastics carriers.

10. Composition according to any of the preceding claims for enhancing the hair follicle-stimulating effect of melatonin through the addition of ginkgo biloba and biotin.

11. Use of a composition according to any of the preceding claims for producing a composition for promoting hair growth.

12. Use of a composition according to any of the preceding claims for producing a composition for the prevention or/and treatment of alopecia.

13. Use according to claim 12 for the treatment of alopecia in men.

14. Use according to claim 12 for the treatment of alopecia in women.

15. Use according to any of claims 11 to 14,
**characterized in that**
from 0.001 mg to 10 mg of melatonin are administered per application.

16. Use according to claim 15,
**characterized in that**
from 0.01 mg to 1 mg of melatonin are administered per application.

## Revendications

1. Composition sous forme de formulation topique à utiliser dans les cheveux, **caractérisée en ce qu'**elle contient comme principes actifs
(a) de la mélatonine ou un dérivé de celle-ci
(b) du ginkgo biloba et
(c) de la biotine,
le dérivé de mélatonine étant choisi parmi la 5-méthoxytryptamine, le 5-méthoxytryptophane, le 5-méthoxytryptophol, l'acide 5-méthoxy-indol-3-acétique et la 6-hydroxy-mélatonine et des sels, esters et complexes physiologiquement acceptables de ceux-ci.

2. Composition selon la revendication 1,
**caractérisée en ce**
**qu'**elle contient du ginkgo biloba sous forme d'extrait sec et/ou un ou plusieurs composants de celui-ci.

3. Composition selon l'une quelconque des revendications précédentes,
**caractérisée en ce**
**que** la concentration de chacun des principes actifs se situe respectivement dans une plage entre 0,0001 % (poids) et 1 % (poids) par rapport au poids total de la composition.

4. Composition selon l'une quelconque des revendications précédentes,
**caractérisée en ce**
**que** les principes actifs se présentent sous forme dissoute et/ou dispersée dans un système de support liquide, semi-solide ou solide.

5. Composition selon l'une quelconque des revendications précédentes,
**caractérisée en ce**
**qu'**elle permet une distribution contrôlée d'au moins l'un des principes actifs.

6. Composition selon l'une quelconque des revendications précédentes pour des utilisations pharmaceutiques et/ou cosmétiques.

7. Composition selon la revendication 1,
**caractérisée en ce**
**que** l'absorption transdermique d'au moins l'un des principes actifs peut être contrôlée.

8. Composition selon l'une quelconque des revendications précédentes sous forme de solution, de suspension, d'émulsion, de micro-émulsion, de nano-système, de crème, de gel, de lotion, de spray, de mousse ou de pommade.

9. Composition selon l'une quelconque des revendications précédentes, dans un système de conditionnement ou d'utilisation choisi parmi des ampoules unidoses en matière plastique ou en verre, des tubes, des flacons, des flacons pulvérisateurs, des emplâtres, des éponges et des supports en textile ou en matière plastique.

10. Composition selon l'une quelconque des revendications précédentes, pour renforcer l'action stimulant le follicule pileux de la mélatonine, par l'addition de Ginkgo biloba et de biotine.

11. Utilisation d'une composition selon l'une quelconque des revendications précédentes pour la production d'un agent destiné à renforcer la croissance des cheveux.

12. Utilisation d'une composition selon l'une quelconque des revendications précédentes pour la production d'un agent destiné à la prévention et/ou au traitement de l'alopécie.

13. Utilisation selon la revendication 12, pour le traitement de l'alopécie chez l'homme.

14. Utilisation selon la revendication 12, pour le traitement de l'alopécie chez la femme.

15. Utilisation selon l'une des revendications 11 à 14,
**caractérisée en ce**
**que** par application, 0,001 mg à 10 mg de mélatonine sont administrés.

16. Utilisation selon la revendication 15,
**caractérisée en ce**
**que**, par application, 0,01 mg à 1 mg de mélatonine sont administrés.
